# FASCICULE DE BREVET EUROPEEN

(11) **EP 2 575 789 B1**
(45) Date de publication et mention de la délivrance du brevet: **23.10.2019**
(21) Numéro de dépôt: 11725693.3
(22) Date de dépôt: 07.06.2011
(51) Int. Cl.: A61K 9/50, A61K 31/00, A61K 9/20, A61K 9/48

(54) **MICROGRANULES ET MICROCOMPRIMES RESISTANTS AU DETOURNEMENT**
ABLENKUNGSRESISTENTE MIKROKÖRNER UND MIKROTABLETTEN
DIVERSION-RESISTANT MICROGRANULES AND MICROTABLETS

(30) Priorité: 20.07.2010 FR 1055921; 07.06.2010 FR 1054465
(43) Date de publication de la demande: 10.04.2013
(73) Titulaire: Ethypharm, 92210 Saint-Cloud (FR)
(72) Inventeur: BILLOET, Vincent, F-76300 Sotteville Les Rouen (FR)
(74) Mandataire: Regimbeau
(86) Numéro de dépôt international: PCT/EP2011/059410
(87) Numéro de publication internationale: WO 2011/154414

(56) Documents cités:
- WO-A1-97/12605
- WO-A1-2006/056712
- FR-A1- 2 881 652
- FR-A1- 2 892 937
- US-A1- 2003 068 371
- US-A1- 2007 224 129

## Description

L'invention a pour objet l'utilisation d'une forme pharmaceutique orale à base de microgranules et/ou de microcomprimés pour réduire l'utilisation abusive d'au moins un principe actif qu'elle contient.

Les principes actifs considérés sont des principes actifs pharmaceutiques, par exemple ceux classés dans la catégorie des produits stupéfiants qui peuvent donc provoquer chez l'homme une dépendance. Ces principes actifs sont ceux qui peuvent donner lieu à un emploi abusif. Plus particulièrement, les principes actifs considérés sont les principes actifs analgésiques.

L'utilisation abusive d'une forme pharmaceutique solide pour usage oral se rencontre dans le cas de comportement addictif, par exemple chez les toxicomanes.

Il est connu, qu'en général l'effet produisant la dépendance est plus important en cas d'administration parentérale ou nasale que dans le cas d'une administration orale. Les personnes cherchant à détourner l'utilisation d'une forme pharmaceutique orale solide contenant au moins un principe actif créant une dépendance vont généralement tenter soit de réduire cette forme pharmaceutique solide à l'état de poudre pulvérulente pouvant être inhalée ou avalée, soit d'extraire le principe actif dans un solvant, par exemple de l'eau, de manière à pouvoir l'injecter à l'aide d'une seringue.

L'obtention d'une forme liquide injectable à partir d'une forme pharmaceutique orale solide, passe par une étape d'extraction aqueuse ou organique du principe actif visé. Cette extraction est généralement précédée d'un broyage.

L'inhalation ou l'injection sont généralement utilisées par les toxicomanes car ce sont des modes de détournement d'usage qui permettent d'accentuer les effets recherchés du principe actif du fait d'une absorption dans l'organisme plus rapide que dans le cas d'une prise orale.

Il existe donc un problème de santé publique lié au mésusage des médicaments, et en particulier des médicaments oraux solides, et plus spécialement dans le cas des principes actifs analgésiques.

Le but visé par la présente invention est de prévenir le détournement d'une forme pharmaceutique orale à base de microgranules et/ou de microcomprimés tels que décrit dans la revendication 1 contenant au moins un principe actif pouvant créer une dépendance, un agent gélifiant et un activateur de la gélification. L'agent gélifiant et l'activateur ne sont mis en contact qu'en cas de détournement par écrasement. Ce couple d'excipients choisi judicieusement confère à la formulation une viscosité telle que ladite formulation ne peut pas être administrée par injection ou ne libère pas le principe actif rapidement en formant un gel au contact de la muqueuse en cas d'administration par voie nasale.

L'état de la technique décrit plusieurs tentatives de réponse au détournement d'un médicament et en particulier de médicaments contenant un opioïde.

Le brevet FR2361914 décrit des comprimés de propiram contenant des dérivés de méthylcellulose, substances gonflant dans l'eau, pour empêcher l'extraction par l'eau du principe actif. L'écrasement de tels comprimés permet une extraction aisée du principe actif.

La demande PCT WO2008150526 décrit une forme pharmaceutique orale contenant un agent gélifiant, un lipide et une substance active.

L'agent gélifiant peut être l'acide hyaluronique, la carboxyméthylcellulose, la gomme guar ou une combinaison de gomme guar et de gomme xanthane.

Le lipide peut être une huile animale, végétale ou minérale liquide ou solide.

En présence d'eau, un gel visqueux se forme de manière à prévenir l'extraction de la substance active.

La demande PCT WO2006056712 décrit une forme pharmaceutique contenant un agent mottant, un agent viscosifiant et des billes insolubles en milieux aqueux ou hydroalcoolique, incompressibles et inertes.

L'agent mottant peut être une huile ou une cire.

L'agent viscosifiant peut être un polyacide acrylique, un polyalkylène glycol, une polyvinylpyrrolidone, une gélatine, un polysaccharide, comme l'alginate de sodium, les pectines, les guars, les xanthanes, les carraghénanes, les gellanes et les dérivés de la cellulose (e.g. hydroxypropylméthylcellulose, méthylcellulose, hydroxyéthylcellulose, carboxyméthylcellulose), et leurs mélanges.

L'état de la technique est aussi décrit dans les documents suivants :
FR 2 892 937 A1 (FLAMEL TECHNOLOGIES SA [FR]) 11 mai 2007
FR 2 881 652 A1 (FLAMEL TECHNOLOGIES SA [FR]) 11 août 2006
US 2007/224129 A1 (GUIMBERTEAU FLORENCE [FR] ET AL) 27 septembre 2007
US 2003/068371 A1 (OSHLACK BENJAMIN [US] ET AL) 10 avril 2003
WO 97/12605 A1 (EURO CELTIQUE SA [LU]; KRISHNAMURTHY THINNAYAM NAGANA [CA]) 10 avril 1997

Les solutions apportées par les documents de l'état de l'art reposent sur l'utilisation d'une substance gélifiante en présence ou non d'une substance hydrophobe pour réduire l'extraction du principe actif.

Cependant ces solutions ne sont pas complètement satisfaisantes car l'augmentation de la viscosité du broyat des formes pharmaceutiques solides et donc la capacité à récupérer ou non le principe actif dépend uniquement des propriétés intrinsèques viscosifiantes des agents gélifiants et de leur quantité dans la formulation.

Un objectif essentiel de la présente invention est de proposer une forme pharmaceutique comprenant un principe actif dont l'utilisation abusive par écrasement puis injection ou inhalation n'est pas possible.

Cet objectif est atteint par une forme pharmaceutique orale à base de deux populations de microgranules ou de microcomprimés d'aspect extérieur identique, la première population (1) contenant au moins un agent gélifiant et au moins un principe actif pouvant créer une dépendance et la seconde population (2), dépourvue de principe actif et d'agent gélifiant, contenant un activateur de gélification.

On peut encore envisager que la première population de microgranules ou de microcomprimés contienne au moins un activateur de gélification et au moins un principe actif et que la seconde population de microgranules ou de microcomprimés dépourvue de principe actif et d'activateur de gélification contienne un agent gélifiant.

L'obtention d'une gélification réduisant la possibilité de détournement d'usage est obtenue non pas en augmentant la quantité de gélifiant dans la formulation, tel que décrit par exemple dans l'art antérieur, mais en optimisant l'utilisation des propriétés viscosifiantes d'un gel.

Lorsque la forme pharmaceutique de l'invention libère ces microgranules et/ou microcomprimés dans l'organisme, en particulier dans le suc gastrique, les populations (1) et (2) ne sont pas en contact. Il n'y a donc pas gélification de la forme pharmaceutique. La population (1) est libérée dans l'organisme comme une forme pharmaceutique à part entière : elle peut donc être formulée comme toute formulation pharmaceutique connue de l'homme du métier à libération immédiate ou prolongée.

Il est connu de l'art antérieur que l'ajout d'un gélifiant peut modifier la libération prolongée d'une forme pharmaceutique.

Contrairement à l'art antérieur, la nature du gélifiant et la quantité du gélifiant ne modifie pas le profile de libération du principe actif de la population (1) libérée dans l'organisme.

Définitions au sens du présent exposé de l'invention :

### Forme pharmaceutique

On entend par « forme pharmaceutique orale » toute forme pharmaceutique orale susceptible d'être préparée à partir de microgranules et/ou de microcomprimés comprenant le principe actif, notamment une suspension, un sirop, un comprimé, une gélule.

### Support neutre

On entend par "support neutre" ou "noyau neutre" ou encore plus simplement "neutre", des supports inertes sphériques ou quasi-sphériques de taille comprise entre 50 µm et 3mm et préférentiellement entre 100 et 1000 µm, tels que ceux habituellement utilisés dans l'industrie pharmaceutique comme support de base de principes actifs pour la constitution de microgranules par exemple.

### Microgranules

Les microgranules de la présente invention se rapportent à des unités galéniques sphériques, constituées en leur centre d'un support neutre, recouvert d'au moins une couche.

Les microgranules de la présente invention peuvent également être obtenus par un procédé en lui-même connu tel que, par exemple, l'extrusion-sphéronisation, la granulation humide ou à chaud.

### Microcomprimés

Les microcomprimés de la présente invention se rapportent à des unités galéniques résultant de la compression de poudres ou de granules et ont une taille inférieure à 10 mm.

### Libération prolongée

Dans la présente demande, on utilisera le terme libération prolongée pour désigner un profil de libération du principe actif modifié par rapport à celui qu'aurait présenté le principe actif seul dans un système à libération immédiate comme défini par la Pharmacopée Européenne (quantité de principe actif libérée en 45 minutes au moins égale à 75%, Ph. Eur., 6ème édition 2.9.3.)

### Activateur de gélification

Dans la présente demande, on utilisera le terme « activateur de gélification » pour désigner un composé capable d'augmenter le pouvoir de gélification d'un agent gélifiant donné. Par exemple pourront être utilisés les activateurs de gélification suivants : un agent ionique, tels que des cations polyvalents, généralement bi- ou trivalents, tels que l'ion calcium ou l'ion aluminium qui se fixent en certains sites précis de chaînes macromoléculaires, que l'on appelle sites de fixation ou sites de réticulation, formant ainsi des pontages entre ces chaînes. Les gels ainsi formés sont parfois appelés "gels ionotropes". Parmi ces gels, on peut citer les alginates, les pectines, les carraghénanes, la carboxyméthylcellulose et les chitosanes.

Dans le cas de l'alginate, par exemple, les cations polyvalents, tels que l'ion calcium Ca²⁺, forment des pontages en certains sites précis des chaînes polysaccharidiques, correspondant à des séquences polyglucuroniques, réalisant ainsi un gel.

Dans la présente demande, on utilisera le terme activateur pour désigner aussi un modificateur de pH, par exemple un acide ou une base organique ou inorganique capable de modifier sensiblement le pH d'une solution / suspension aqueuse.

Dans la présente demande, on pourra aussi utiliser le terme activateur de gélification pour désigner un composé d'origine minérale.

Par exemple, dans le cas de la gomme xanthane, l'utilisation de dérivé d'argile, type Veegum®, va augmenter de façon synergique la viscosité de la suspension obtenue une fois le mélange dispersé dans l'eau.

### DESCRIPTION DETAILLEE DE L'INVENTION

L'invention concerne une forme pharmaceutique orale à base de microgranules et/ou de microcomprimés comprenant deux populations de microgranules ou de microcomprimés d'aspect extérieur identique, la première population (1) comprenant au moins un principe actif pouvant créer une dépendance et au moins un agent gélifiant, et la seconde population (2), dépourvue de principe actif et d'agent gélifiant, comprenant au moins un activateur de gélification.

Il peut être envisagé également que la première population (1) de microgranules ou de microcomprimés comprenne au moins un principe actif pouvant créer une dépendance et au moins un activateur de gélification, et que la seconde population (2) de microgranules ou de microcomprimés, dépourvue de principe actif et d'activateur de gélification, comprenne au moins un agent gélifiant.

Selon un premier mode de réalisation de l'invention, la forme pharmaceutique orale consiste en deux populations de microgranules ou en deux populations de microcomprimés.

Plus précisément, la première population de microgranules peut comprendre un support neutre et au moins une couche de montage comprenant au moins un principe actif et éventuellement un agent liant pharmaceutiquement acceptable et un agent gélifiant présent soit dans la couche de montage soit dans une couche séparée.

La seconde population de microgranules peut comprendre un support neutre et au moins une couche de montage comprenant au moins un activateur de gélification et éventuellement un agent liant pharmaceutiquement acceptable.

En cas de détournement de la forme pharmaceutique orale, par exemple par écrasement des microgranules en présence d'eau ou d'un autre solvant par exemple l'alcool, la première population de microgranules libère le principe actif et l'agent gélifiant et de façon concomitante et sans ségrégation possible, la seconde population de microgranules libère le déclencheur de gélification de manière à diminuer ou de préférence empêcher la solubilisation du principe actif contenu dans les microgranules.

Optionnellement, la première population de microgranules comporte un enrobage à base d'au moins un polymère hydrophobe ou hydrosoluble. Le polymère hydrophobe empêche la libération immédiate du principe actif et retarde la libération (libération prolongée). La nature et la quantité de gélifiant ne modifient pas le comportement du polymère hydrophobe à libération prolongée. La couche d'enrobage comprend, optionnellement, au moins un plastifiant et optionnellement au moins un agent tensio-actif. Selon un autre mode de réalisation de l'invention, la forme pharmaceutique orale consiste en deux populations de microgranules. Le polymère hydrophobe est présent en quantité suffisante pour empêcher la libération immédiate du principe actif lors de condition normale d'utilisation de la forme pharmaceutique orale selon l'invention.

De façon avantageuse, le support neutre est constitué soit de saccharose et d'amidon soit d'au moins un excipient de nature hydrophobe choisi parmi : la cellulose, les dérivés de la cellulose (cellulose microcristalline), les dérivés des phosphates (phosphates de calcium), la silice et les dérivés des silicates (silicate de magnésium, silicate d'aluminium et leurs mélanges), ou encore tout type d'excipient apte à former des sphères de taille régulière, par exemple l'acide tartrique.

Selon un autre mode de réalisation de l'invention, la forme pharmaceutique orale comprend des microcomprimés.

Les différents procédés de fabrication des comprimés, par exemple par compression directe ou par compression après granulation par voie humide ou par voie sèche, sont présentés dans « Remington's pharmaceutical Sciences, 16 ème Ed, 1980, Mack Publ. Co. of Easton, PA, USA».

Les microcomprimés conformes à l'invention peuvent être préparés par compression directe ou par tout autre procédé approprié.

De façon avantageuse, les microcomprimés selon la présente invention sont obtenus par mélange de poudres soumis à une homogénéisation dans un mélangeur à sec. Le mélange est ensuite soumis à une force de compression qui confère au comprimé résultant une dureté satisfaisante permettant l'industrialisation de sa fabrication et sa manipulation dans des conditions normales sans précautions opératoires particulières.

Selon un aspect particulier de l'invention, la forme pharmaceutique orale consiste en deux populations de microcomprimés d'aspect extérieur identique, la première population de microcomprimés comprenant au moins un principe actif et un agent gélifiant, et la seconde population de microcomprimés, dépourvue de principe actif et d'agent gélifiant, comprenant au moins un activateur de gélification.

Il peut être envisagé également que la forme pharmaceutique orale consiste en deux populations de microcomprimés d'aspect extérieur identique, la première population de microcomprimés comprenant au moins un principe actif et un activateur de gélification, et la seconde population de microcomprimés, dépourvue de principe actif et d'activateur de gélification, comprenant au moins un agent gélifiant.

En cas de détournement de la forme pharmaceutique orale, par exemple par écrasement des microcomprimés en présence d'eau ou d'un autre solvant par exemple l'alcool, la première population de microcomprimés libère le principe actif et l'agent gélifiant et de façon concomitante et sans ségrégation possible, la seconde population de microcomprimés libère le déclencheur de gélification de manière à diminuer ou de préférence empêcher la solubilisation du principe actif contenu dans les microcomprimés.

Selon un autre mode de réalisation de l'invention, la forme pharmaceutique orale consiste en deux populations de microcomprimés d'aspect extérieur identique.

La première population de microcomprimés comprend au moins un principe actif et un activateur de gélification,

La seconde population de microcomprimés comprend au moins un agent gélifiant.Chaque microcomprimé selon la présente invention comprend au moins un diluant pharmaceutiquement acceptable.

Optionnellement, la première population de microcomprimé comporte un enrobage à base d'au moins un polymère hydrophobe. Le polymère hydrophobe empêche la libération immédiate du principe actif. La couche d'enrobage comprend, optionnellement, au moins un plastifiant et optionnellement au moins un agent tensio-actif

De façon avantageuse, l'agent gélifiant est présent dans la forme pharmaceutique finale à raison de 1 à 100 mg, plus préférentiellement de 1 à 50 mg et encore plus préférentiellement de 1 à 30 mg.

De façon avantageuse, l'activateur de la gélification est présent dans la forme pharmaceutique finale à raison de 1 à 100 mg, plus préférentiellement de 1 à 50 mg et encore plus préférentiellement de 1 à 30 mg.

De façon avantageuse également, un polymère hydrosoluble et un polymère non hydrosoluble peuvent entrer dans la composition d'une couche d'enrobage du microgranule ou du microcomprimé. Ce polymère hydrosoluble est choisi dans le groupe comprenant notamment la polyvinylpyrrolidone, l'hydroxypropylméthyl cellulose et leurs mélanges, et le polymère non hydrosoluble est choisi dans le groupe comprenant notamment les résines acryliques et/ou méthacryliques, les polymères cellulosiques, les polymères vinyliques et leurs mélanges.

De préférence, le ou les principes actifs sont intégrés dans la couche active du microgranule en association avec un agent liant pharmaceutiquement acceptable, tel que ceux habituellement utilisés dans l'industrie pharmaceutique pour la fixation de principes actifs à la surface de supports neutres. Ainsi, la méthode de fixation de la couche active décrite dans le brevet EP 1 200 071 peut tout à fait être employée pour la fixation de la couche active dans le cadre de la présente invention.

De façon préférée, la couche active des microgranules conformes à l'invention est appliquée par pulvérisation d'une dispersion de principe actif dans un solvant (appelée dispersion de montage). Avantageusement, cette dispersion contient également l'agent liant.

Parmi les agents liants pharmaceutiquement acceptables, on utilisera préférentiellement des agents liants de nature hydrophile et notamment des dérivés de la cellulose tels que l'HPMC, en particulier les grades Pharmacoat® 603 et Pharmacoat® 606, des dérivés de la polyvinylpyrrolidone, en particulier le grade PVP K 30 et également des dérivés du polyéthylene glycol, en particulier le polyéthylene glycol dont le poids moléculaire vaut entre 3000 et 7000, tels que le PEG4000 et le PEG6000 notamment, et leurs mélanges.

Le solvant de la dispersion de montage pulvérisé doit être adapté au principe actif ou au mélange de principes actifs employés. Ainsi, on pourra par exemple utiliser l'eau, des solvants organiques, parmi eux l'éthanol ou des solutions hydro-alcooliques de diverses concentrations pour la réalisation de la solution ou suspension à la base de la couche active.

Un agent tensioactif peut être ajouté à la phase de montage pour améliorer la solubilité du principe actif ou stabiliser la suspension de montage. L'agent tensioactif est utilisé en quantités de 0 à 50% et préférentiellement entre 0 et 20%. Parmi les tensioactifs utilisables, peuvent être cités les sels alcalins ou alcalinoterreux des acides gras (dont les stéarates, de préférence de calcium, de magnésium, d'aluminium ou de zinc), le sodium dodécyl sulfate et le docusate de sodium étant préférés, les huiles polyoxyéthylénées, de préférence l'huile de ricin hydrogénée polyoxyéthylénée, les copolymères polyoxyéthylène-polyoxypropylène, les esters de sorbitan polyoxyéthylénés (=polysorbates), les dérivés de l'huile de ricin polyoxyéthylénés, les stéarylfumarates, de préférence de sodium, le béhénate de glycérol, le chlorure de benzalkonium, le bromure d'acétyltriméthyl ammonium, l'alcool cétylique et leurs mélanges.

Dans la mesure du possible, il est préférable d'utiliser des solvants non toxiques et facilement éliminables par évaporation lors du séchage afin qu'il n'en subsiste aucune trace dans les microgranules.

De façon avantageuse, les agents gélifiants sont choisis dans les groupes de polymères suivants :
- les polyacides acryliques et leurs dérivés, type carbopol® (appelé Carbomer ou carboxy polyméthylène)
- les polyoxyéthylènes (POE), et/ou
- les alcools polyvinyliques (APV)
- les polyvinylpyrrolidones (PVP), et/ou
- les gélatines, et/ou
- les dérivés de la cellulose (e.g. hydroxypropylméthylcellulose, méthylcellulose, hydroxyéthylcellulose, carboxyméthylcellulose), et/ou
- les polysaccharides, de préférence dans le sous-groupe comprenant : les alginates, en particulier l'alginate de sodium, les pectines, les guars, les xanthanes, les carraghénanes, les gellanes et - et leurs mélanges.

De façon avantageuse, lorsque l'activateur de gélification est un alginate par exemple le Protanal® 120 ou 200, l'activateur de gélification est un cation divalent tel que l'ion Ca²⁺, l'ion Ba²⁺, l'ion Zn²⁺, l'ion Cu²⁺, l'ion Mg²⁺

Lorsque l'agent gélifiant est le Carbopol®, l'activateur de gélification est un agent alcalinisant tel que l'hydroxyde de potassium, le bicarbonate de sodium, l'hydroxyde de sodium, le phosphate de sodium ou une amine organique telle que la triéthanolamine.

De manière générale, l'activateur de gélification utilisé en association avec l'agent gélifiant permet la formation de pontages en certains sites des chaînes polymériques de l'agent gélifiant ou encore permet le renforcement du réseau polymérique.

De façon avantageuse, selon l'invention le couple agent gélifiant/ activateur de gélification est choisi parmi les associations suivantes :
- carboxy polyméthylène (carbopol®)/ bicarbonate de sodium,
- alginate/ ion Ca²⁺,
- alginate/ un dérivé d'argile, type Veegum® (silicates de magnésium et d'aluminium),
- gomme xanthane/ un dérivé d'argile, type Veegum® (silicates de magnésium et d'aluminium),
- alcools polyvinyliques/ ion Cu²⁺,
- pectines/ ion Ca²⁺,
- carboxyméthylcellulose/ Al²⁺,
- gomme gellane/ Ca²⁺,
- K-carraghénane/ K⁺,
- I-carraghénane/ Ca²⁺.

Les différentes populations de microgranules ou de microcomprimés selon la présente invention ont toutes la même apparence extérieure, de manière à ce que les microgranules ou les microcomprimés contenant le principe actif soient physiquement indiscernables des microparticules ou des microcomprimés comprenant l'agent de gélification ou l'activateur de gélification. Ceci dans le but que la séparation des différentes populations de microgranules ou de microcomprimés, en vue d'un détournement d'usage, soit rendue impossible.

De façon avantageuse, l'enrobage permettant d'indifférencier les différentes populations de microgranules ou de microcomprimés est constitué par un film polymère pouvant être coloré, par exemple l'Opadry®.

De façon avantageuse, l'enrobage peut permettre de contrôler la libération du principe actif contenu dans les microgranules ou les microcomprimés. L'enrobage contient alors un polymère hydrophobe empêchant la libération immédiate du principe actif en quantité comprise entre 50% à 100%, de préférence entre 50% et 90%, encore préférentiellement entre 50% et 80%, 50% et 70%, ou 50% et 60%, de la masse sèche de ladite couche d'enrobage.

Le taux d'enrobage représente le rapport de la quantité de masse sèche constituant l'enrobage assurant une libération prolongée du principe actif sur la masse totale du microgranule ou du microcomprimé avant enrobage (en masse sèche). Le taux d'enrobage est compris entre 0,1% à 50% m/m, de préférence, de 2% à 30% m/m, et, plus préférentiellement encore, de 5% à 30%. Ou autrement dit, le rapport entre la masse de vernis sec (= polymère et éventuels additifs en masse sèche) constituant l'enrobage empêchant la libération immédiate du principe actif sur la masse totale du microgranule ou du microcomprimé avant enrobage (en masse sèche) est compris entre 0,1% à 50% m/m, de préférence, de 2% à 30% m/m, et, plus préférentiellement encore, de 5% à 30%.

Les polymères utilisés pour assurer une libération prolongée du principe actif sont des polymères de nature hydrophobe, de préférence, sélectionnés dans le groupe de produits suivants : les dérivés non hydrosolubles de la cellulose, les dérivés de (co)polymères (méth)acryliques, les dérivés vinyliques et leurs mélanges.

Plus préférentiellement, le ou les polymère(s) hydrophobe(s) empêchant la libération immédiate du principe actif est (sont) sélectionné(s) dans le groupe de produits suivants : l'éthylcellulose, l'acétate butyrate de cellulose, l'acétate de cellulose, les copolymères ammonio-méthacrylates type A et type B vendus sous le nom commercial Eudragit®, notamment l'Eudragit® RS 30D, l'Eudragit NE 30D, l'Eudragit® RL 30D, l'Eudragit® RS PO et l'Eudragit® RL PO de la famille des poly(éthyl acrylate, méthyl méthacrylate, triméthylamonioéthyl méthacrylate), les polyvinylacétates et leurs mélanges.

Un agent plastifiant peut être ajouté à la dispersion d'enrobage à raison de 0% à 50% m/m, de préférence, de 2% à 25% m/m, en masse sèche de polymère d'enrobage.

L'agent plastifiant est sélectionné notamment dans le groupe de produits suivants : le glycérol et ses esters, de préférence dans le sous-groupe suivant : les triglycérides à chaînes moyennes, les glycérides acétylés, glycéryl-mono-stéarate, glycéryl-triacétate, glycéryl-tributyrate, les phtalates, de préférence dans le sous-groupe suivant : dibutylphtalate, diéthylphtalate, diméthylphtalate, dioctylphtalate, les citrates, de préférence dans le sous-groupe suivant : acétyltributylcitrate, acétyltriéthylcitrate, tributylcitrate, triéthylcitrate, les sébaçates, de préférence dans le sous-groupe suivant : diéthylsébaçate, dibutylsébaçate, les adipates, les azélates, les benzoates, le chlorobutanol, les polyéthylène glycols, les huiles végétales, les fumarates, de préférence le diéthylfumarate, les malates, de préférence le diéthylmalate, les oxalates, de préférence le diéthyloxalate, les succinates ; de préférence le dibutylsuccinate, les butyrates, les esters de l'alcool cétylique, les malonates, de préférence le diéthylmalonate, l'huile de ricin (celle-ci étant particulièrement préférée), et leurs mélanges.

Plus préférentiellement, l'agent plastifiant est sélectionné dans le groupe de produits suivants : les monoglycérides acétylés notamment le Myvacet® 9-45, le triéthylcitrate (TEC), le dibutylsébacate, la triacétine et leurs mélanges.

L'agent tensioactif est optionnellement présent dans l'enrobage à raison de 0 à 30% m/m, de préférence de 0 à 20% m/m, et, plus préférentiellement encore, de 5 à 15% de la masse sèche de plastifiant. L'agent tensioactif est de préférence sélectionné dans le groupe de produits suivants : les sels alcalins ou alcalinoterreux des acides gras (de préférence les stéarates, de préférence de calcium, de magnésium, d'aluminium ou de zinc), le sodium dodécyl sulfate et le docusate de sodium étant préférés, les huiles polyoxyéthylénées, de préférence l'huile de ricin hydrogénée polyoxyéthylénée, les copolymères polyoxyéthylène-polyoxypropylène, les esters de sorbitan polyoxyéthylénés (= polysorbates), les dérivés de l'huile de ricin polyoxyéthylénés, les stéarylfumarates, de préférence de sodium, le béhénate de glycérol, le chlorure de benzalkonium, le bromure d'acétyltriméthyl ammonium, l'alcool cétylique et leurs mélanges.

Une charge inerte peut être présente dans l'enrobage à raison de 0 à 50% m/m, de préférence de 0 à 20% m/m, et, plus préférentiellement encore, de 5 à 20% de la masse sèche du polymère d'enrobage.

La charge inerte uniformément répartie dans l'enrobage est choisie dans le groupe comprenant notamment le talc, la silice colloïdale anhydre, le stéarate de magnésium, le monostéarate de glycérol et leurs mélanges.

La forme pharmaceutique orale à base de microgranules selon la présente invention peut se présenter sous forme de sachet ou d'emballage unidose de microgranules ou de comprimés obtenus à partir de microgranules, de comprimé obtenu à partir de microgranules, ou de gélule contenant des microgranules.

La forme pharmaceutique orale à base de microcomprimés selon la présente invention peut se présenter sous forme de sachet ou d'emballage unidose de microcomprimés ou de gélule contenant des microcomprimés.

La forme pharmaceutique orale à base de microgranules ou de microcomprimés selon la présente invention comprend des microgranules ou des microcomprimés soit à libération modifiée du principe actif soit à libération immédiate du principe actif.

Pour préparer les microcomprimés selon la présente invention on utilisera les excipients connus de l'homme du métier.

Par exemple, les sucres et les carbohydrates sont couramment utilisés comme liants et délitants dans la formulation de comprimés en raison de leur goût agréable.

Le lactose directement compressible est l'un des excipients parmi les plus utilisés en compression directe : il est cependant incompatible avec certains principes actifs.

L'amidon directement compressible (ou amidon prégélatinisé) subit un traitement chimique et mécanique pour éviter l'agrégation des grains d'amidon. Il est constitué de 5% amylose, 15% amylopectine et 80% amidon non modifié. Il est utilisé comme liant (sous forme d'empois), comme diluant ou comme désintégrant.

Le saccharose directement compressible contient entre 95 et 98% de saccharose et un additif comme l'amidon, la maltodextrine, le sucre inverti ou un lubrifiant. Il est utilisé comme liant et surtout comme diluant.

Parmi les autres excipients pour compression directe figurent le mannitol, la cellulose microcristalline et le phosphate dicalcique. On a également développé des granulés pour compression directe présentant une bonne fluidité à base de fructose, de lactitol ou de xylitol, ils sont préparés par atomisation ou par agglomération.

Les unités galéniques selon l'invention peuvent être pelliculées, soit pour améliorer leur aspect, soit pour masquer la couleur, soit pour protéger le principe actif de la lumière, de l'humidité ou de l'oxygène de l'air.

### Le principe actif

La couche active constituant les microgranules conformes à l'invention comprend au moins un principe actif pharmaceutique qui peut être de toute nature.

Les microgranules ou les microcomprimés selon la présente invention peuvent comprendre en tant que principe actif, les principes actifs agissant sur le système nerveux central.

Les principes actifs agissant sur le système nerveux central sont de préférence choisis parmi les anti-épileptiques, les anti-parkinsonniens, les psycho-stimulants, les psychotropes, les antidépresseurs, les anxiolytiques et les anti-psychotiques par exemple.

Les analgésiques peuvent être choisis parmi les analgésiques non opiacés, opiacés faibles, opioïdes mixtes, morphiniques ou spasmodiques.

Plus précisément encore, le principe actif mis en oeuvre est choisi parmi les composés suivants : Alfentanil, Alphaméthylfentanyl, Buprénorphine, Codéine, Dextropropoxyphène, Dihydrocodéine, Hydrocodone, Hydromorphone, Méthadone, Morphine, Oxycodone, Oxymorphone, Sufentanil, et Tramadol, les sels pharmaceutiquement acceptables de ces composés et les mélanges de ces composés et/ou de leurs sels.

### Le procédé de préparation des microgranules

Les microgranules de la présente peuvent être préparés par le procédé qui comprend les étapes suivantes :- l'introduction de supports sphériques neutres dans une enceinte réactionnelle à lit fluidisé, - la pulvérisation sur ces supports sphériques neutres d'au moins un principe actif en solution ou en suspension dans un solvant organique et/ou aqueux additionné d'au moins un polymère hydrosoluble ou non hydrosoluble, - la pulvérisation d'une suspension d'enrobage comprenant au moins un polymère hydrophobe sur les particules enrobées obtenues à l'étape précédente,- éventuellement, le séchage des microgranules médicamenteux ainsi obtenus.

### Préparation de la dispersion de montage

L'étape dite de montage de la couche active conformément à la présente invention permet d'obtenir des microgranules dont la teneur en actif est à la fois précise et uniforme.

La dispersion dite de montage est la dispersion dans laquelle le ou les principes actifs vont être dissous ou mis en suspension (dispersés) et qui va être pulvérisée à la surface des microgranules. Cette dispersion contient avantageusement un agent liant conventionnel dissous également.

### Montage de la couche active

Le principe actif est appliqué sur les granules de façon conventionnelle par pulvérisation, en lit fluidisé ou en turbine perforée par exemple. D'une manière générale, ce procédé repose sur la pulvérisation simultanée au travers d'une buse, du ou des principes actifs et éventuellement d'un liant qui sont dissous ou dispersés dans la solution de montage, ce qui garantit pour cette étape du procédé une parfaite homogénéité de teneur.

Le temps nécessaire au montage est très variable et dépend de la quantité d'actif à pulvériser et de sa solubilité dans la solution de montage. De manière générale il est compris entre 1 et 10 heures.

A l'issue de l'étape de montage, les microgranules sont séchés en lit fluidisé ou en turbine perforée puis tamisés.

Selon une variante de la présente invention la couche d'agent gélifiant peut-être appliquée soit directement sur le neutre (granule), soit sur la couche active après séchage de cette dernière.

### Application de l'activateur de gélification

L'activateur de gélification est appliqué sur les neutres (granules) de façon conventionnelle par pulvérisation, en lit fluidisé ou en turbine perforée par exemple.

### Enrobage des microgranules

Le polymère d'enrobage est appliqué sur les microgranules précédents de façon conventionnelle par pulvérisation, en lit fluidisé ou en turbine perforée par exemple. D'une manière générale, ce procédé repose sur la pulvérisation simultanée au travers d'une buse, du ou des polymères d'enrobage et éventuellement d'un plastifiant et/ou d'un agent tensioactif et/ou d'une charge inerte qui sont dissous ou dispersés dans un solvant adapté.

Une solution organique de polymère peut être utilisée pour l'enrobage : dans ce cas, le procédé consiste en la pulvérisation de la solution et un séchage dans le même équipement.

Si le véhicule est l'eau, une dispersion aqueuse de polymère est utilisée, un plastifiant doit être ajouté pour améliorer la qualité de l'enrobage. Le procédé consiste alors en la pulvérisation de la dispersion, un séchage dans le même équipement et, si nécessaire, une étape de maturation du film d'enrobage (aussi appelée curing) qui permet l'obtention d'un film homogène et uniforme. Le curing peut être réalisé en lit fluidisé, en turbine perforée ou en étuve par exemple.

Le temps nécessaire à l'enrobage est très variable et dépend de la quantité de polymère à pulvériser. De manière générale il est compris entre 1 et 10 heures.

A l'issue de l'étape d'enrobage, les microgranules sont séchés en lit fluidisé puis tamisés.

Les exemples suivants sont donnés à titre illustratif de la présente invention. Ils ne constituent en aucun cas une limite des possibilités.

### EXEMPLES

### Exemple 1a: Microgranules de sulfate de morphine à libération prolongée résistants au détournement d'usage

### a) Préparation de la population 1, incluant le principe actif et le gélifiant

Le principe actif utilisé est le sulfate de morphine.

Les noyaux neutres utilisés sont des sphères de sucre (Neutres #30 NPPHARM). La taille de ces supports est de l'ordre de 400 à 600 µm.

Le gélifiant utilisé est un dérivé de polyacides acryliques, type Carbopol® 971 ou 974.

L'agent liant utilisé est l'hydroxypropylméthylcellulose 603 (HPMC 603)

Le Carbopol® est solubilisé dans l'eau, puis l'HPMC 603 et enfin le sulfate de morphine sont ajoutés à cette solution aqueuse, constituant la solution de montage. La solution de montage est pulvérisée en lit fluidisé (Glatt), de même que la suspension d'enrobage conférant les propriétés de libération prolongée à cette population de microgranules.

Formules des différentes populations 1 (incluant le principe actif et le gélifiant) :

| | Prototype A | | Prototype B | | Prototype C | |
|---|---|---|---|---|---|---|
| | Quantité (g) | Composition centésimale* | Quantité (g) | Composition centésimale* | Quantité (g) | Composition centésimale* |
| Noyaux neutres | 750,0g | 31,6% | 750,0g | 30,0% | 750,0g | 31,6% |
| Carbopol 971® | - | - | - | | 375,0g | 15,8% |
| Carbopol 974® | 375,0g | 15,8% | 500,0g | 20,0% | - | - |
| HPMC 603 | 250,0g | 10,5% | 250,0g | 10,0% | 250,0g | 10,5% |
| Sulfate de morphine | 750,0g | 31,6% | 750,0g | 30,0% | 750,0g | 31,6% |
| Enrobage LP | 250,0g | 10,5% | 250,0g | 10,0% | 250,0g | 10,5% |
| Total masse sèche | 2375,0g | 100,0% | 2500,0g | 100,0% | 2375,0g | 100,0% |

| | | | | | | |
|---|---|---|---|---|---|---|
| *Exprimé en matières sèches | | | | | | |

### b) préparation de la population 2 incluant l'activateur de gélification : le bicarbonate de sodium

Les noyaux neutres utilisés sont des sphères de sucre (Neutres #30 NPPHARM). La taille de ces supports est de l'ordre de 400 à 600 µm.

L'agent liant utilisé est le polyoxyéthylèneglycol 6000 (PEG 6000)

L'activateur de gélification est le bicarbonate de sodium.

Le PEG 6000, puis le bicarbonate de sodium sont solubilisés dans l'eau, constituant la solution de montage. La solution de montage est pulvérisée en lit fluidisé (Glatt), de même que la suspension d'enrobage permettant d'éviter que le bicarbonate de sodium ne soit libéré dans le cadre d'une utilisation normale.

Formules des différentes populations 2 (incluant l'activateur) :

| | Prototype A | | Prototype B | | Prototype C | |
|---|---|---|---|---|---|---|
| | Quantité (g) | Composition centésimale* | Quantité (g) | Composition centésimale* | Quantité (g) | Composition centésimale* |
| Noyaux neutres | 750,0g | 25,0% | 750,0g | 25,0% | 750,0g | 25,0% |
| PEG 6000 | 750,0g | 25,0% | 750,0g | 25,0% | 750,0g | 25,0% |
| Bicarbonate de Na | 750,0g | 25,0% | 750,0g | 25,0% | 750,0g | 25,0% |
| Enrobage LP | 750,0g | 25,0% | 750,0g | 25,0% | 750,0g | 25,0% |
| Total masse sèche | 3000,0g | 100,0% | 3000,0g | 100,0% | 3000,0g | 100,0% |

| | | | | | | |
|---|---|---|---|---|---|---|
| *Exprimé en matières sèches | | | | | | |

### c) Mélange des populations 1 et 2

Les populations 1 et 2 de chaque prototype A, B et C sont ensuite respectivement mélangées avant mise en gélules selon les proportions suivantes :

| | Prototype A | | Prototype B | | Prototype C | |
|---|---|---|---|---|---|---|
| | Quantité (g) | Composition centésimale* | Quantité (g) | Composition centésimale* | Quantité (g) | Composition centésimale* |
| Population 1 (avec actif) | 1900,0g | 67,9% | 2000,0g | 62,5% | 1900,0g | 67,9% |
| Population 2 (avec activateur) | 900,0g | 32,1% | 1200,0g | 37,5% | 900,0g | 32,1% |
| Total masse sèche | 2800,0g | 100,0% | 3200,0g | 100,0% | 2800,0g | 100,0% |

### Exemple1b : Microgranules de sulfate de morphine à libération prolongée résistants au détournement d'usage

### a) Préparation de la population 1, incluant le principe actif et le gélifiant

Le principe actif utilisé est le sulfate de morphine.

Les noyaux neutres utilisés sont des sphères de sucre (Neutres #30 NPPHARM). La taille de ces supports est de l'ordre de 400 à 600 µm.

Le gélifiant utilisé est un dérivé d'alginate, type Protanal® 120 ou 200.

L'agent liant utilisé est l'hydroxypropylméthylcellulose 603 (HPMC 603)

Le Protanal® est solubilisé dans l'eau, puis l'HPMC 603 et enfin le sulfate de morphine sont ajoutés à cette solution aqueuse, constituant la solution de montage. La solution de montage est pulvérisée en lit fluidisé (Glatt), de même que la suspension d'enrobage conférant les propriétés de libération prolongée à cette population de microgranules.

**Formules des différentes populations 1 (incluant le principe actif et le gélifiant) :**

| | Prototype A | | Prototype B | | Prototype C | |
|---|---|---|---|---|---|---|
| | Quantité (g) | Composition centésimale* | Quantité (g) | Composition centésimale* | Quantité (g) | Composition centésimale* |
| Noyaux neutres | 750,0g | 31,6% | 750,0g | 31,6% | 750,0g | 33,4% |
| Protanal 120® | - | - | 375,0g | 15,8% | 250,0g | 11,1% |
| Protanal 200® | 375,0g | 15,8% | - | - | - | - |
| HPMC 603 | 250,0g | 10,5% | 250,0g | 10,5% | 250,0g | 11,1% |
| Sulfate de morphine | 750,0g | 31,6% | 750,0g | 31,6% | 750,0g | 33,3% |
| Enrobage LP | 250,0g | 10,5% | 250,0g | 10,5% | 250,0g | 11,1% |
| Total masse sèche | 2375,0g | 100,0% | 2375,0g | 100,0% | 2250,0g | 100,0% |

| | | | | | | |
|---|---|---|---|---|---|---|
| * Exprimé en matières sèches | | | | | | |

### b) préparation de la population 2 incluant l'activateur de gélification : le chlorure de calcium

Les noyaux neutres utilisés sont des sphères de sucre (Neutres #30 NPPHARM). La taille de ces supports est de l'ordre de 400 à 600 µm.

L'agent liant utilisé est le polyoxyéthylèneglycol 6000 (PEG 6000)
L'activateur est le chlorure de calcium.

Le PEG 6000, puis le chlorure de calcium sont solubilisés dans l'eau, constituant la solution de montage. La solution de montage est pulvérisée en lit fluidisée (Glatt), de même que la suspension d'enrobage permettant d'éviter que le chlorure de calcium ne soit libéré dans le cadre d'une utilisation normale.

**Formules des différentes populations 2 (incluant l'activateur) :**

| | Prototype A | | Prototype B | | Prototype C | |
|---|---|---|---|---|---|---|
| | Quantité (g) | Composition centésimale* | Quantité (g) | Composition centésimale* | Quantité (g) | Composition centésimale* |
| Noyaux neutres | 750,0g | 25,0% | 750,0g | 25,0% | 750,0g | 25,0% |
| PEG 6000 | 750,0g | 25,0% | 750,0g | 25,0% | 750,0g | 25,0% |
| Chlorure de Calcium | 750,0g | 25,0% | 750,0g | 25,0% | 750,0g | 25,0% |
| Enrobage LP | 750,0g | 25,0% | 750,0g | 25,0% | 750,0g | 25,0% |
| Total masse sèche | 3000,0g | 100,0% | 3000,0g | 100,0% | 3000,0g | 100,0% |

| | | | | | | |
|---|---|---|---|---|---|---|
| * Exprimé en matières sèches | | | | | | |

### c) Mélange des populations 1 et 2

Les populations 1 et 2 de chaque prototype A, B et C sont ensuite respectivement mélangées avant mise en gélules selon les proportions suivantes :

| | Prototype A | | Prototype B | | Prototype C | |
|---|---|---|---|---|---|---|
| | Quantité (g) | Composition centésimale* | Quantité (g) | Composition centésimale* | Quantité (g) | Composition centésimale* |
| Population 1 (avec actif) | 1900,0g | 86,4% | 1900,0g | 86,4% | 1800,0g | 85,7% |
| Population 2 (avec activateur) | 300,0g | 13,6% | 300,0g | 13,6% | 300,0g | 14,3% |
| Total masse sèche | 2200,0g | 100,0% | 2200,0g | 100,0% | 2100,0g | 100,0% |

### Exemple1c: Microgranules de sulfate de morphine à libération prolongée résistants au détournement d'usage

### a) Préparation de la population 1, incluant le principe actif et le gélifiant

Le principe actif utilisé est le sulfate de morphine.

Les noyaux neutres utilisés sont des sphères de sucre (Neutres #30 NPPHARM). La taille de ces supports est de l'ordre de 400 à 600 µm.

Le gélifiant utilisé est une gomme xanthane, type Vanzan®.

L'agent liant utilisé est l'hydroxypropylméthylcellulose 603 (HPMC 603)

La gomme xanthane est solubilisée dans l'eau, puis l'HPMC 603 et enfin le sulfate de morphine sont ajoutés à cette solution aqueuse, constituant la solution de montage. La solution de montage est pulvérisée en lit fluidisée (Glatt), de même que la suspension d'enrobage conférant les propriétés de libération prolongée à cette population de microgranules.

**Formules des différentes populations 1 (incluant le principe actif et le gélifiant) :**

| | Prototype A | |
|---|---|---|
| | Quantité (g) | Composition centésimale* |
| Noyaux neutres | 750,0g | 35,2% |
| Vanzan® | 125,0g | 5,9% |
| HPMC 603 | 250,0g | 11,8% |
| Sulfate de morphine | 750,0g | 35,3% |
| Enrobage LP | 250,0g | 11,8% |
| Total masse sèche | 2125,0g | 100,0% |

| | | |
|---|---|---|
| * Exprimé en matières sèches | | |

### b) préparation de la population 2 incluant l'activateur de gélification : le silicate de magnésium et d'aluminium

Les noyaux neutres utilisés sont des sphères de sucre (Neutres #30 NPPHARM). La taille de ces supports est de l'ordre de 400 à 600 µm.

L'agent liant utilisé est le polyoxyéthylèneglycol 6000 (PEG 6000)

L'activateur est le silicate de magnésium et d'aluminium.

Le PEG 6000, puis le silicate de magnésium et d'aluminium sont dispersés dans l'eau, constituant la suspension de montage. La suspension de montage est pulvérisée en lit fluidisée (Glatt), de même que la suspension d'enrobage permettant d'éviter que le silicate de magnésium et d'aluminium ne soit libéré dans le cadre d'une utilisation normale.

**Formules de la population 2 (incluant l'activateur) :**

| | Prototype A | |
|---|---|---|
| | Quantité (g) | Composition centésimale* |
| Noyaux neutres | 750,0g | 20,0% |
| PEG 6000 | 750,0g | 20,0% |
| silicate de magnésium et d'aluminium | 1500,0g | 40,0% |
| Enrobage LP | 750,0g | 20,0% |
| Total masse sèche | 3750,0g | 100,0% |

| | | |
|---|---|---|
| • Exprimé en matières sèches | | |

### c) Mélange des populations 1 et 2

Les populations 1 et 2 sont ensuite respectivement mélangées avant mise en gélules selon les proportions suivantes :

| | Prototype A | |
|---|---|---|
| | Quantité (g) | Composition centésimale* |
| Population 1 (avec actif) | 1700,0g | 68,0% |
| Population 2 (avec activateur) | 800,0g | 32,0% |
| Total masse sèche | 2500,0g | 100,0% |

### Exemple 2 : Microcomprimés de sulfate de morphine à libération prolongée résistants au détournement d'usage

### a) Préparation de la population 1

### La population 1 incluant le principe actif et le gélifiant

Le principe actif utilisé est le sulfate de morphine.

On introduit dans un mélangeur le principe actif, la cellulose microcristalline (Avicel PH102), le Protanal 120. On prépare un mélange homogène.

On arrête le mélangeur et on ajoute le stéarate de magnésium et on poursuit l'opération de mélange pendant 1 à 5 min suivant la masse du mélange.

La machine à comprimer est équipée de poinçons adaptés à la fabrication de microcomprimés et elle est réglée pour obtenir des microcomprimés de masse de l'ordre de 30mg.

### b) Préparation de la population 2

### La population 2 incluant l'activateur de gélification

On introduit dans un mélangeur la cellulose microcristalline (Avicel PH102), le Chlorure de calcium. On prépare un mélange homogène.

On arrête le mélangeur et on ajoute le stéarate de magnésium et on poursuit l'opération de mélange pendant 1 à 5 min suivant la masse du mélange.

La machine à comprimer est équipée de poinçons adaptés à la fabrication de microcomprimés et elle est réglée pour obtenir des microcomprimés de masse de l'ordre de 30mg.

**Formules des différentes populations :**

| **Population 1** | **%** | **g** |
|---|---|---|
| Protanal 120 | 16.67% | 100.0 |
| Morphine sulfate | 33.33% | 200.0 |
| Avicel PH102 | 32.92% | 197.5 |
| Stéarate de Mg | 0.42% | 2.5 |
| Enrobage LP | 16.67% | 100.0 |
| Total | 100.01 % | 600.0 |
| | | |

| **Population 2** | **%** | **g** |
|---|---|---|
| Chlorure de calcium | 41.67% | 250.0 |
| Avicel PH102 | 41.25% | 247.5 |
| Stéarate de Mg | 0.42% | 2.5 |
| Enrobage LP | 16.67% | 100.0 |
| Total | 100.00% | 600.0 |

**Suspension d'enrobage LP :**

| | Quantité |
|---|---|
| Eudragit L30D-55 | 63.33% |
| Talc | 12.5 % |
| Triéthylcitrate | 20.83 % |
| Silice collodiale | 3.33% |
| Total masse sèche | 100% |

La suspension d'enrobage conférant les propriétés de libération prolongée à cette population de microcomprimés est pulvérisée en lit fluidisé (Glatt)

### b) Mélange des populations 1 et 2

Les populations 1 et 2 sont ensuite respectivement mélangées avant mise en gélules selon les proportions suivantes :

| **Mélange** | % | g |
|---|---|---|
| Population 1 | 71.43% | 600.0 |
| Population 2 | 28.57% | 240.0 |
| Total | 100.00% | 840.0 |

### Exemple 3 : Test de broyage de microgranules réalisés selon l'exemple 1a

| | **Population 1** | **Population 2** |
|---|---|---|
| Neutres | 60,00 mg | 40,00 mg |
| PEG6000 | | 40,00 mg |
| HPMC | 20,00 mg | |
| NaHCO3 | | 40,00 mg |
| Carbopol 974 | 40,00 mg | |
| Carbopol 971 | | |
| ex: Morphine | 60,00 mg | |
| Enrobage LP | 20,00 mg | |
| | | |
| Total | 200,00 mg | 120,00 mg |
| | | |
| Total pour 1 gélule | | 320,00 mg |

Les microgranules d'une gélule sont écrasés puis délayés dans 1 ml d'eau. La solution résultante est un gel compact impropre à l'injection IV.

Lorsque la même expérience est répétée avec 2, 5 ou 10 ml, la solution résultante est un gel visqueux impropre à l'injection IV.

### Exemple 4 : L'Exemple 2 a été réitéré avec les 4 formulations suivantes

**Exemple 4a :**

| **Population 1** | pesée (g) | mg/cp | % |
|---|---|---|---|
| Hydromorphone HCl | 64,00 | 4,80 | 16,0 |
| Carbopol 71G | 111,30 | 8,35 | 27,8 |
| Kolidon SR | 222,70 | 16,70 | 55,7 |
| Stéarate de Mg | 2,00 | 0,15 | 0,5 |
| Total | 400,00 | 30,00 | 100,0 |

| **Population 2** | pesée (g) | mg/cp | % |
|---|---|---|---|
| Bicarbonate de sodium | 160,00 | 12,00 | 40,0 |
| Avicel pH200 | 236,00 | 17,70 | 59,0 |
| Syloid | 2,00 | 0,15 | 0,5 |
| Stéarate de Mg | 2,00 | 0,15 | 0,5 |
| Total | 400,00 | 30,0 | 100,0 |

La forme pharmaceutique finale pour cet exemple est un mélange des microcomprimés des populations 1 et 2 avec par exemple le ratio suivant : 5 microcomprimés de la population 1 et 4 microcomprimés de la population 2. Ceci correspond à une dose de 24mg d'hydromorphone HCI par unité de prise. La quantité de gélifiant (Carbopol 71G) est de 42mg et celle d'activateur de la gélification (bicarbonate de sodium) de 48mg. Ce mélange de microcomprimés peut être mis en gélule ou en emballage unidose.

**Exemple 4b :**

| **Population 1** | pesée (g) | mg/cp | % |
|---|---|---|---|
| Hydromorphone HCl | 64,00 | 4,80 | 16,0 |
| Protanal LF 200 | 111,30 | 8,35 | 27,8 |
| Kolidon SR | 222,70 | 16,70 | 55,7 |
| Stéarate de Mg | 2,00 | 0,15 | 0,5 |
| Total | 400,00 | 30,00 | 100,0 |

| **Population 2** | pesée (g) | mg/cmp | % |
|---|---|---|---|
| Chlorure de Calcium | 160,00 | 12,00 | 40,0 |
| Avicel pH200 | 236,00 | 17,70 | 59,0 |
| Syloid | 2,00 | 0,15 | 0,5 |
| Stéarate de Mg | 2,00 | 0,15 | 0,5 |
| Total | 400,00 | 30,00 | 100,0 |

La forme pharmaceutique finale pour cet exemple est un mélange des microcomprimés des populations 1 et 2 avec par exemple le ratio suivant : 7 microcomprimés de la population 1 et 1 microcomprimé de la population 2. Ceci correspond à une dose de 34mg d'hydromorphone HCI par unité de prise. La quantité de gélifiant (Protanal LF 200) est de 58mg et celle d'activateur de la gélification (chlorure de Calcium) de 12mg. Ce mélange de microcomprimés peut être mis en gélule ou en emballage unidose.

**Exemple 4c :**

| **Population 1** | pesée (g) | mg/cp | % |
|---|---|---|---|
| Oxvcodone HCI | 64,00 | 4,80 | 16,0 |
| Carbopol 71G | 111,30 | 8,35 | 27,8 |
| Avicel PH200 | 222,70 | 16,70 | 55,7 |
| Stéarate de Mg | 2,00 | 0,15 | 0,5 |
| Total | 400,00 | 30,00 | 100,0 |

| **Population 2** | pesée (g) | mg/cp | % |
|---|---|---|---|
| Bicarbonate de sodium | 160,00 | 12,00 | 40,0 |
| Avicel PH200 | 236,00 | 17,70 | 59,0 |
| Syloid | 2,00 | 0,15 | 0,5 |
| Stéarate de Mg | 2,00 | 0,15 | 0,5 |
| Total | 400,00 | 30,0 | 100,0 |

La forme pharmaceutique finale pour cet exemple est un mélange des microcomprimés des populations 1 et 2 avec par exemple le ratio suivant : 9 microcomprimés de la population 1 et 8 microcomprimés de la population 2. Ceci correspond à une dose de 43mg d'oxycodone HCI par unité de prise. La quantité de gélifiant (Carbopol 71G) est de 75mg et celle d'activateur de la gélification (bicarbonate de sodium) de 96mg. Ce mélange de microcomprimés peut être mis en emballage unidose ou en sachet.

**Exemple 4d :**

| **Population 1** | pesée (g) | mg/cp | % |
|---|---|---|---|
| Oxvcodone HCI | 64,00 | 4,80 | 16,0 |
| Gomme Xanthane | 111,30 | 8,35 | 27,8 |
| Kolidon SR | 222,70 | 16,70 | 55,7 |
| Stéarate de Mg | 2,00 | 0,15 | 0,5 |
| Total | 400,00 | 30,00 | 100,0 |

| **Population 2** | Pesée (g) | mg/cp | Quantité (%) |
|---|---|---|---|
| Veegum NF-F | 160,00 | 12,00 | 40,0 |
| Syloid 244 | 4,00 | 0,30 | 1,0 |
| Avicel Ph200 | 234,00 | 17,55 | 58,5 |
| Stéarate de Mg | 2,00 | 0,15 | 0,5 |
| Total | 400,00 | 30,00 | 100,0 |

La forme pharmaceutique finale pour cet exemple est un mélange des microcomprimés des populations 1 et 2 avec par exemple le ratio suivant : 2 microcomprimés de la population 1 et 8 microcomprimés de la population 2. Ceci correspond à une dose de 9,6mg d'oxycodone HCI par unité de prise. La quantité de gélifiant (Gomme xanthane) est de 16,7mg et celle d'activateur de la gélification (Veegum NF-F) de 96mg. Ce mélange de microcomprimés peut être mis en emballage unidose ou en sachet.

### Exemple 5 : Essais d'extraction réalisés sur les formes pharmaceutiques des exemples 4.

La méthode utilisée est la suivante : les micro-comprimés de la forme pharmaceutique finale sont broyés pendant 2 minutes dans un mortier à l'aide d'un pilon afin de les réduire en fine poudre. Cette poudre est ensuite transvasée dans des béchers de 20 mL dans lequel se trouve un barreau aimanté, 10 mL des différents solvants sont ajoutés et l'agitation mise en route pendant 5 minutes exactement. Puis les échantillons sont mis à filtrer sur filtre papier dans un tube en verre pendant 1h exactement. Si aucun filtrat n'est passé, l'échantillon est filtré à l'aide d'une seringue équipée d'un filtre en fibre de verre de porosité 1µm (GF/B). Le filtrat obtenu est dilué au 1/10e dans HCI 0,1N. Dans le cas où aucun filtrat n'est obtenu, il est conclu que 0% du principe actif n'a pu être extrait. Enfin, les échantillons sont passés en HPLC dans le but de déterminer le pourcentage de principe actif extrait.

Concernant l'Oxycodone HCl, l'analyse HPLC s'effectue avec une colonne C18 en phase inverse avec détection UV à 280 nm. Le solvant d'élution est un mélange d'eau, d'acide heptanesulfonique et d'acétonitrile.

Concernant l'Hydromorphone HCl, l'analyse HPLC s'effectue avec une colonne C18 en phase inverse avec détection UV à 230 nm. Le solvant d'élution est un mélange d'eau, d'acide heptanesulfonique et d'acétonitrile.

Les tableaux ci-dessous regroupent les résultats d'extraction des différentes formulations des exemples 4.

Les pourcentages présentés correspondent au pourcentage de principe actif passé dans le filtrat après broyage et extraction dans les différents solvants testés. Cela correspond au pourcentage de la dose initiale contenue dans la forme pharmaceutique qu'une personne voulant la détourner pourrait s'injecter.

Dans la colonne de gauche de chaque tableau est présenté le % récupérable lorsque seuls les microcomprimés avec principe actif et gélifiant (population 1) sont utilisés pour l'extraction. Dans la colonne de droite, à la population 1 sont ajoutés des microcomprimés de la population 2. Les quantités utilisées pour faire ces essais sont celles présentées dans l'exemple 4.

L'intérêt de tester en parallèle avec et sans la population 2 permet de mettre en évidence son intérêt dans le cadre de la présente invention.

### Exemple 5a : résultats obtenus avec la forme pharmaceutique finale de l'exemple 4a.

| Gélifiant | Carbopol (42mg) | |
|---|---|---|
| Activateur de gélification | - | Bicarbonate de Na (48mg) |

| Principe actif | Hydromorphone (24mg) LP | |
|---|---|---|
| Solvant | % récupéré | % récupéré |
| Eau | 33,3 % | 0,0 % |
| Eau + 40% EtOH | 25,4 % | 6,7 % |

### Exemple 5b : résultats obtenus avec la forme pharmaceutique finale de l'exemple 4b.

| Gélifiant | Protanal (58mg) | |
|---|---|---|
| Activateur de gélification | - | CaCl2 (12mg) |

| Principe actif | Hydromorphone (34mg) | |
|---|---|---|
| Solvant | % récupéré | % récupéré |
| Eau | 29,1% | 0,0% |
| Eau + 40% EtOH | 29,1% | 17,2% |

### Exemple 5c : résultats obtenus avec la forme pharmaceutique finale de l'exemple 4c.

| Gélifiant | Carbopol (75mg) | |
|---|---|---|
| Activateur de gélification | - | Bicarbonate de Na (96mg) |

| Principe actif | Oxycodone (43mg) IR | |
|---|---|---|
| Solvant | % récupéré | % récupéré |
| Eau | 37,2% | 0,0% |
| HCL 0,1N | 19,6% | 16,0% |

### Exemple 5d : résultats obtenus avec la forme pharmaceutique finale de l'exemple 4d.

| Gélifiant | Gomme Xanthane (16,7mg) | |
|---|---|---|
| Activateur de gélification | - | Veegum (96mg) |

| Principe actif | Oxycodone (9,6mg) | |
|---|---|---|
| Solvant | % récupéré | % récupéré |
| Eau | 0,0 % | 0,0 % |
| Eau + 40% EtOH | 0,0 % | 0,0 % |
| Ethanol | 57,2 % | 11,4 % |
| HCl 0,1N | 15,9 % | 0,7 % |
| Vinaigre cuisine | 7,3 % | 0,5 % |

### Exemple 6 : Essais de dissolution réalisés sur les formes pharmaceutiques des exemples 4.

Dans le but de montrer que différents types de profils de libération sont envisageables, des essais de dissolution ont été réalisés sur les différents formules des exemples 4, sur les microcomprimés non écrasés.

Les conditions de dissolution sont les suivantes :
Pour le principe actif hydromorphone HCl, les essais de dissolution ont été conduits en bain à dissolution conformes à la Pharmacopée Européenne, les agitateurs de chaque vase étant équipés de pales tournant à 100 tours/min. Le milieu de dissolution utilisé est composé de 500mL de tampon pH 6,8 à 37°C. L'analyse des différents points de dissolution se faisant par HPLC à 230nm selon la méthode décrite dans l'exemple 5.
Pour le principe actif oxycodone HCl, les essais de dissolution ont été conduits en bain à dissolution conformes à la Pharmacopée Européenne, les agitateurs de chaque vase étant équipés de paniers tournant à 100 tours/min. Le milieu de dissolution utilisé est composé de 900mL de tampon pH 1,2 à 37°C. L'analyse des différents points de dissolution se faisant par HPLC à 230nm selon la méthode décrite dans l'exemple 5.

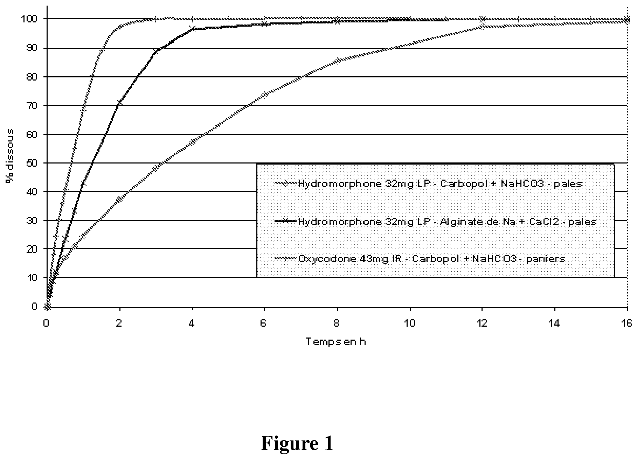

Les résultats obtenus sont présentés sur la Figure 1. Les profils de dissolution sont différents les uns des autres, montrant que malgré une gélification intense lorsque la forme est détournée, lorsque les comprimés sont intacts, il est possible d'obtenir des profils de libération plus ou moins rapides.

## Revendications

1. Forme pharmaceutique orale à base de microgranules et/ou de microcomprimés comprenant deux populations de microgranules ou de microcomprimés de même apparence extérieure, la première population comprenant au moins un principe actif pouvant créer une dépendance et un agent gélifiant, et la deuxième population, dépourvue de principe actif et d'agent gélifiant, comprenant au moins un activateur de gélification,
dans laquelle l'activateur de gélification, en association avec l'agent gélifiant, permet la formation de pontages en certains sites des chaînes polymériques de l'agent gélifiant ou/et permet le renforcement du réseau polymérique de l'agent gélifiant.

2. Forme pharmaceutique selon la revendication 1, constituée de microgranules ou de microcomprimés.

3. Forme pharmaceutique selon la revendication 2, **caractérisée par** une première population de microgranules comprenant un support neutre et au moins une couche de montage comprenant au moins un principe actif et éventuellement un agent liant pharmaceutiquement acceptable et un agent gélifiant présent soit dans la couche de montage soit dans une couche séparée, et une seconde population de microgranules comprenant un support neutre et au moins une couche de montage comprenant au moins un activateur de gélification.

4. Forme pharmaceutique selon l'une quelconque des revendications 1 à 3, **caractérisée en ce que** les agents gélifiants sont choisis parmi les polyacides acryliques et leurs dérivés, les polyoxyéthylènes, les alcools polyvinyliques, les polyvinylpyrrolidones (PVP), les gélatines, les dérivés de la cellulose tels que hydroxypropylméthylcellulose, méthylcellulose, hydroxyéthylcellulose, carboxyméthylcellulose, les polysaccharides, de préférence dans le sous-groupe comprenant : l'alginate de sodium, les pectines, les guars, les xanthanes, les carraghénanes, les gellanes et leurs mélanges.

5. Forme pharmaceutique selon l'une quelconque des revendications 1 à 4, **caractérisée en ce que** le couple agent gélifiant/ activateur de gélification est choisi parmi carboxy polyméthylène / bicarbonate de sodium, alginate/ ion Ca²⁺, alginate/ silicates de magnésium et d'aluminium et gomme xanthane/ silicates de magnésium et d'aluminium.

6. Forme pharmaceutique selon l'une quelconque des revendications 1 à 5, **caractérisée en ce que** l'agent gélifiant est présent dans l'unité galénique à raison de 1 à 100 mg, plus préférentiellement de 1 à 50 mg et encore plus préférentiellement de 1 à 30 mg.

7. Forme pharmaceutique selon l'une quelconque des revendications 1 à 6, **caractérisée en ce que** l'activateur de la gélification est présent dans l'unité galénique à raison de 1 à 100 mg, plus préférentiellement de 1 à 50 mg et encore plus préférentiellement de 1 à 30 mg.

8. Forme pharmaceutique selon l'une quelconque des revendications 1 à 7, **caractérisée en ce que** les microgranules et/ou les microcomprimés comportent un enrobage à base d'au moins un polymère hydrophobe pour empêcher la libération immédiate du principe actif.

9. Forme pharmaceutique selon la revendication 8, **caractérisée en ce que** le polymère hydrophobe est choisi parmi l'éthylcellulose, l'acétate butyrate de cellulose, l'acétate de cellulose, les copolymères ammonio-méthacrylates type A et type B vendus sous le nom commercial Eudragit®, notamment l'Eudragit® RS 30D, l'Eudragit NE 30D, l'Eudragit® RL 30D, l'Eudragit® RS PO et l'Eudragit® RL PO de la famille des poly(éthyl acrylate, méthyl méthacrylate, triméthylamonioéthyl méthacrylate), les polyvinylacétates et leurs mélanges.

10. Forme pharmaceutique selon la revendication 9, **caractérisée en ce que** l'enrobage comprend au moins un plastifiant.

11. Forme pharmaceutique selon l'une quelconque des revendications 1 à 10, **caractérisée en ce que** le principe actif est choisi parmi les analgésiques et notamment les analgésiques non opiacés, opiacés faibles, opioïdes mixtes, morphiniques ou spasmodiques, notamment l'hydrocodone, l'hydromorphone, la morphine, l'oxycodone, l'oxymorphone, le tramadol et leurs dérivés.

12. Forme pharmaceutique selon la revendication 1, dans laquelle les microgranules ou les microcomprimés sont conditionnés sous forme de gélule.

13. Forme pharmaceutique selon la revendication 1, dans laquelle les microgranules ou les microcomprimés sont conditionnés sous forme de comprimé multiparticulaire.

14. Forme pharmaceutique selon la revendication 1, dans laquelle les microgranules ou les microcomprimés sont conditionnés en emballage unidose.

## Patentansprüche

1. Orale pharmazeutische Form auf Mikrokörnchen- und / oder Mikrotabletten-Basis, umfassend zwei Mikrokörnchen- oder Mikrotabletten-Populationen desselben äußeren Erscheinungsbildes, wobei die erste Population wenigstens ein Wirkstoffprinzip umfasst, das eine Abhängigkeit und ein Geliermittel herstellen kann, und die zweite Population ohne Wirkstoffprinzip und Geliermittel wenigstens einen Gelieraktivator umfasst,
bei der der Gelieraktivator in Verbindung mit dem Geliermittel die Bildung von Überbrückungen an bestimmten Stellen der Polymerketten des Geliermittels zulässt und / oder die Verstärkung des Polymernetzes des Geliermittels zulässt.

2. Pharmazeutische Form gemäß Anspruch 1, gebildet aus Mikrokörnchen oder Mikrotabletten.

3. Pharmazeutische Form gemäß Anspruch 2, **gekennzeichnet durch** eine erste Mikrokörnchen-Population, umfassend einen neutralen Träger und wenigstens eine Montageschicht, umfassend wenigstens ein Wirkstoffprinzip und eventuell ein pharmazeutisch akzeptables Bindemittel und ein Geliermittel, das entweder in der Montageschicht oder in einer gesonderten Schicht vorhanden ist, und eine zweite Mikrokörnchen-Population, umfassend einen neutralen Träger und wenigstens eine Montageschicht, umfassend wenigstens einen Gelieraktivator.

4. Pharmazeutische Form gemäß irgendeinem der Ansprüche 1 bis 3, **dadurch gekennzeichnet, dass** die Geliermittel aus Acrylpolysäure und deren Derivaten, Polyoxyethylenen, Polyvinylalkoholen, Polyvinylpyrrolidonen (PVP), Gelatinen, den Derivaten der Zellulose, wie z. B. Hydroxypropylmethylzellulose, Methylzellulose, Hydroxyethylzellulose, Carboxymethylzellulose, Polysacchariden, bevorzugt aus der Untergruppe, umfassend: Natriumalginat, Pektine, Guare, Xanthane, Carraghenane, Gellane und deren Mischungen ausgewählt sind.

5. Pharmazeutische Form gemäß irgendeinem der Ansprüche 1 bis 4, **dadurch gekennzeichnet, dass** das Paar Geliermittel / Gelieraktivator aus Carboxy-Polymethylen / Natrium-Bikarbonat, Alginat / Ion Ca²⁺, Alginat / Magnesium- und Aluminiumsilikaten und Xanthangummi / Magnesium- und Aluminiumalginaten ausgewählt ist.

6. Pharmazeutische Form gemäß irgendeinem der Ansprüche 1 bis 5, **dadurch gekennzeichnet, dass** das Geliermittel in einer galenischen Einheit in einer Menge von 1 bis 100 mg, bevorzugt von 1 bis 50 mg und noch weiter bevorzugt von 1 bis 30 mg vorhanden ist.

7. Pharmazeutische Form gemäß irgendeinem der Ansprüche 1 bis 6, **dadurch gekennzeichnet, dass** das Gelieren in der galenischen Einheit in einer Menge von 1 bis 100 mg, weiter bevorzugt von 1 bis 50 mg und noch weiter bevorzugt von 1 bis 30 mg vorhanden ist.

8. Pharmazeutisch Form gemäß irgendeinem der Ansprüche 1 bis 7, **dadurch gekennzeichnet, dass** die Mikrokörnchen und/oder Mikrotabletten eine Umhüllung auf der Basis von wenigstens einem hydrophoben Polymer umfassen, um die sofortige Freisetzung des Wirkstoffprinzips zu verhindern.

9. Pharmazeutische Form gemäß Anspruch 8, **dadurch gekennzeichnet, dass** das hydrophobe Polymer aus Ethylzellulose, Zellulosebutyrat-Acetat, Zellulose-Acetat, den Kopolymeren Ammonium-Methacrylat vom Typ A und B, die unter dem Handelsnamen Eudragit®, insbesondere Eudragit® RS 30D, Eudragit NE 30D, Eudragit® RL 30D, Eudragit® RS PO und Eudragit® RL PO aus der Familie der Poly-(Ethyl-Acrylat, MethylMethacrylat, Trimethylammoniumethyl-Methacrylat), den Polyvinylacetaten und deren Mischungen verkauft werden.

10. Pharmazeutische Form gemäß Anspruch 9, **dadurch gekennzeichnet, dass** die Umhüllung wenigstens einen Weichmacher umfasst.

11. Pharmazeutische Form gemäß irgendeinem der Ansprüche 1 bis 10, **dadurch gekennzeichnet, dass** das Wirkstoffprinzip aus Schmerzstillern und insbesondere nicht opiumhaltigen, schwach opiumhaltigen, gemischten opioiden, morphiumhaltigen oder krampflösenden Schmerzstillern, insbesondere Hydrocodon, Hydromorphon, Morphin, Oxycodon, Oxymorphon, Tramadol und deren Derivaten ausgewählt ist.

12. Pharmazeutische Form gemäß Anspruch 1, bei der die Mikrokörnchen oder die Mikrotabletten in Form einer Kapsel aufbereitet sind.

13. Pharmazeutische Form gemäß Anspruch 1, bei der die Mikrokörnchen oder die Mikrotabletten in Form einer Multipartikeltablette aufbereitet sind.

14. Pharmazeutische Form gemäß Anspruch 1, bei der die Mikrokörnchen oder die Mikrotabletten in einer Einzeldosis-Verpackung aufbereitet sind.

## Claims

1. Oral dosage form based on microgranules and/or microtablets comprising two populations of microgranules or microtablets with the same external appearance, the first population comprising at least one active principle that could create a dependency and a gelling agent, and the second population, without active principle and gelling agent, comprising at least one gelling activator,
wherein the gelling activator, in association with the gelling agent, enables the formation of bridges at certain sites of the polymeric chains of the gelling agent or/and enables the reinforcement of the polymeric network of the gelling agent.

2. Dosage form according to claim 1, constituted of microgranules or microtablets.

3. Dosage form according to claim 2, **characterised by** a first population of microgranules comprising a neutral support and at least one mounting layer comprising at least one active principle and optionally a pharmaceutically acceptable binder agent and a gelling agent present either in the mounting layer or in a separate layer, and a second population of microgranules comprising a neutral support and at least one mounting layer comprising at least one gelling activator.

4. Dosage form according to any of claims 1 to 3, **characterised in that** the gelling agents are chosen from polyacrylic acids and derivatives thereof, polyoxyethylenes, polyvinyl alcohols, polyvinylpyrrolidones (PVP), gelatines, derivatives of cellulose such as hydroxypropylmethylcellulose, methylcellulose, hydroxyethylcellulose, carboxymethylcellulose, polysaccharides, preferably in the sub-group comprising: sodium alginate, pectins, guars, xanthans, carrageenans, gellans and mixtures thereof.

5. Dosage form according to any of claims 1 to 4, **characterised in that** the gelling agent/gelling activator pair is chosen from carboxy polymethylene/sodium bicarbonate, alginate/Ca²⁺ ion, alginate/magnesium and aluminium silicates and xanthan gum/magnesium and aluminium silicates.

6. Dosage form according to any of claims 1 to 5, **characterised in that** the gelling agent is present in the dosage unit at a rate of 1 to 100 mg, more preferentially from 1 to 50 mg and even more preferentially from 1 to 30 mg.

7. Dosage form according to any of claims 1 to 6, **characterised in that** the gelling activator is present in the dosage unit at a rate of 1 to 100 mg, more preferentially from 1 to 50 mg and even more preferentially from 1 to 30 mg.

8. Dosage form according to any of claims 1 to 7, **characterised in that** the microgranules and/or the microtablets comprise a coating based on at least one hydrophobic polymer to prevent the immediate release of the active principle.

9. Dosage form according to claim 8, **characterised in that** the hydrophobic polymer is chosen from ethylcellulose, cellulose butyrate acetate, cellulose acetate, type A and type B ammonio-methacrylate copolymers sold under the trade name Eudragit®, particularly Eudragit® RS 30D, Eudragit NE 30D, Eudragit® RL 30D, Eudragit® RS PO and Eudragit® RL PO of the family of poly(ethyl acrylate, methyl methacrylate, trimethylamonioethyl methacrylate), polyvinylacetates and mixtures thereof.

10. Dosage form according to claim 9, **characterised in that** the coating comprises at least one plastifier.

11. Dosage form according to any of claims 1 to 10, **characterised in that** the active principle is chosen from analgesics and particularly non-opiate, low opiate analgesics, mixed opioids, morphinics or spasmodics, particularly hydrocodone, hydromorphone, morphine, oxycodone, oxymorphone, tramadol and derivatives thereof.

12. Dosage form according to claim 1, wherein the microgranules or the microtablets are conditioned in capsule form.

13. Dosage form according to claim 1, wherein the microgranules or the microtablets are conditioned in multiparticulate tablet form.

14. Dosage form according to claim 1, wherein the microgranules or the microtablets are conditioned in single dose packaging.
